# EUROPÄISCHE PATENTSCHRIFT

(11) **EP 2 115 423 B1**
(45) Veröffentlichungstag und Bekanntmachung des Hinweises auf die Patenterteilung: **06.11.2013**
(21) Anmeldenummer: 08701255.5
(22) Anmeldetag: 05.01.2008
(51) Int. Cl.: G01N 33/543, G01Q 30/04, G01Q 60/28, G01Q 60/42, B82Y 35/00

(54) **VORRICHTUNG UND VERFAHREN FÜR DIE ERFASSUNG VON KRÄFTEN IM SUBMICRONEWTON-BEREICH**
APPARATUS AND METHOD FOR THE DETECTION OF FORCES IN THE SUB-MICRONEWTON RANGE
DISPOSITIF ET PROCÉDÉ POUR LA DÉTECTION DE FORCES DANS LA PLAGE AU-DESSOUS DU MICRO-NEWTON

(30) Priorität: 05.01.2007 DE 102007001797
(43) Veröffentlichungstag der Anmeldung: 11.11.2009
(73) Patentinhaber: Technische Universität München, 80333 München (DE)
(72) Erfinder: HUGEL, Thorsten, 85748 Garching (DE); GEISLER, Michael, 34323 Malsfeld (DE)
(74) Vertreter: Herrmann, Franz
(86) Internationale Anmeldenummer: PCT/EP2008/050075
(87) Internationale Veröffentlichungsnummer: WO 2008/081044

(56) Entgegenhaltungen:
- DE-A1- 19 631 326
- ECKEL R ET AL: "Identification of binding mechanism in single molecule-DNA complexes" BIOPHYSICAL JOURNAL BIOPHYS. SOC USA, Bd. 85, Nr. 3, September 2003 (2003-09), Seiten 1968-1973, XP002475022 ISSN: 0006-3495
- ROS ROBERT ET AL: "Antigen binding forces of individually addressed single-chain Fv antibody molecules" PROCEEDINGS OF THE NATIONAL ACADEMY OF SCIENCES OF THE UNITED STATES OF AMERICA, Bd. 95, Nr. 13, 23. Juni 1998 (1998-06-23), Seiten 7402-7405, XP002475023 ISSN: 0027-8424 in der Anmeldung erwähnt
- KIENBERGER F ET AL: "Recognition force spectroscopy studies of the NTA-His6 bond" SINGLE MOLECULES WILEY-VCH GERMANY, Bd. 1, Nr. 1, 2000, Seiten 59-65, XP002475024 ISSN: 1438-5163
- FLORIN E-L ET AL: "ADHESION FORCES BETWEEN INDIVIDUAL LIGAND-RECEPTOR PAIRS" SCIENCE, AMERICAN ASSOCIATION FOR THE ADVANCEMENT OF SCIENCE,, US, Bd. 264, 15. April 1994 (1994-04-15), Seiten 415-417, XP002072145 ISSN: 0036-8075 in der Anmeldung erwähnt
- IKAI A: "NANOMECHANICS OF PROTEIN-BASED BIOSTRUCTURES" JAPANESE JOURNAL OF APPLIED PHYSICS, JAPAN SOCIETY OF APPLIED PHYSICS, TOKYO, JP, Bd. 43, Nr. 11A, November 2004 (2004-11), Seiten 7365-7375, XP001229988 ISSN: 0021-4922

## Beschreibung

Die Erfindung betrifft eine Vorrichtung für die Erfassung von Kräften im Submikronewton-Bereich mit:
- einem Messkopf, mit dem eine Relativbewegung bezüglich einer Probenaufnahme ausführbar ist,
- wenigstens einem an den Messkopf angebrachten Sondenelement, durch das eine Haftkraft zwischen einer auf der Probenaufnahme angeordneten Probe und dem Messkopf vermittelbar ist,
- einem an dem Messkopf angebrachten länglichem Träger, an den das Sondenelement angesetzt ist, und mit
- einer Auswerteeinheit, mit der die Relativbewegung zwischen Messkopf und Probenaufnahme detektierbar ist, und durch die ein der Haftung des Sondenelements an der Probe zugeordnetes Kraftplateau erfassbar ist.

Die Erfindung betrifft ferner ein Verfahren für die Erfassung von Kräften im Submikronewton-Bereich.

Eine derartige Vorrichtung und ein derartiges Verfahren sind aus der Veröffentlichung ROS ROBERT et al.: Antigen binding forces of individually addressed single-chain Fv antibody molecules" in: Proceedings of the National Academy of Sciences of the United States of America, Bd. 95, Nr. 13, 23. Juni 1998 bekannt. Bei dem bekannten Verfahren werden fragmentierte Antikörper auf eine Goldoberfläche aufgebracht. Ferner werden zu den Antikörpern passende Antigene über eine PEG-Kette an einem Messkopf angebracht und eine Verbindung zwischen Antigen und Antikörper hergestellt. Anschließend wird die Kraft bestimmt, die benötigt wird, um die Verbindung zwischen Antikörper und Antigen zu lösen. Die mit dem bekannten Verfahren aufgenommenen Kraft-Weg-Kurven zeigen den Verlauf einer Abrisskurve, der für den Bruch einer einzelnen Verbindung charakteristisch ist.

Aus der EP 0 829 722 A2 ist ein Rasterkraftmikroskop oder kurz Kraftmikroskop (= Atomic Force Microscope = AFM) bekannt. Das bekannte Kraftmikroskop weist eine als Messkopf dienende Messspitze auf, die entweder direkt oder nach Aufbringen einer Metall- oder Oxidschicht mit einer oder mehreren Sondenmoleküle beschichtet wird. Die Messspitze wird in Kontakt mit der Oberfläche der zu untersuchenden Probe gebracht und die beim Zurückziehen gemessene Kraft gemessen. Damit können qualitative Unterschiede in der Wechselwirkung zwischen Sondenmolekülen und Oberflächen gemessen werden.

Ein entsprechendes Verfahren zur chemisch differenzierenden Abbildung mittels Rasterkraftmikroskopie ist auch aus der EP 0 727 639 A1 bekannt.

Derartige Kraftmikroskope können sowohl zur Abbildung der Topographie von Oberflächen, als auch zur Messung von Bindungskräften zwischen Antikörpern und Antigenen sowie zur chemisch differenzierten Abbildung genutzt werden. Nähere Informationen hierzu finden sich in der Veröffentlichung FLORIN, E.L., MOY, V.T., GAUB, H.E.: Adhesion forces between individual ligand-receptor pairs, Science, 264, 415 (1994), in der Veröffentlichung DAMMER, U., HEGNER, M. et al.: Specific antigen/antibody interactions measured by force microscopy, Biophys. J. 70, 2437 (1996) sowie in der Veröffentlichung SMITH, D.A., CONNELL, S.D. et al.: Chemical force microscopy: applications in surface characterisation of natural hydroxyapatite, Analytica Chimica Acta 479, 39 (2003).

Die Kraftplateaus in der Kraft-Abstandskurve wurden vor einigen Jahren entdeckt und interpretiert. Erste Experimente dazu sind in der Veröffentlichung CHÂTELLIER, T., SENDEN, J. et al.: Detachment of a single polyelectrolyte chain adsorbed on a charged surface, Europhys. Lett., 41, 303 (1998), in der Veröffentlichung HUGEL, T., GROSHOLZ et al.: Elasticity of Single Polyelectrolyte Chains and their desorption from solid supports studied by AFM based Single Molecule Force Spectroscopy Macromolecules, 34, 1039 (2001) sowie in der Veröffentlichung HUGEL, T., SEITZ, M.: The Study of Molecular Interactions by AFM Force Spectroscopy Macromol. Rapid Commun. 22, 989 (2001) beschrieben. Im Rahmen dieser Experimente wurden Polymere auf einer Oberfläche adsorbiert und dann mit einer funktionalisierten Messspitze heruntergezogen. Hier treten zwei Probleme auf. Zum einen ist die Spitze nach kurzer Zeit nicht mehr zu gebrauchen, da die reaktiven Gruppen abgesättigt sind. Zum anderen müssen mehrere hundert Kraft-Abstandskurven aufgenommen werden, bevor eine reine Einzelmolekülkurve gemessen wird, da nicht bei jeder Messung ein Polymer von der Messspitze aufgenommen wird.

Zur Behebung dieser Probleme wurden einige Sondenmoleküle kovalent an die Messspitze angebunden und dann in Kontakt mit der Oberfläche gebracht. Nach einer bestimmten Zeit wurde die Spitze wieder von der Oberfläche weggezogen und die zur Desorption nötige Gleichgewichtskraft gemessen. Dieser Zyklus konnte oft mehrer Dutzend Male wiederholt werden. Experimente dieser Art sind in der Veröffentlichung SEITZ, M., FRIEDSAM, C., et al.: Probing Surfaces with Single Polymers ChemPhysChem. 4, 986 (2003) und FRIEDSAM, C., DEL CAMPO BÉCARES, A. et al.: Polymer Functionalized AFM tips for Long-Term Measurements in Single-Molecule Force Spectroscopy, ChemPhysChem. 5, 388 (2004) beschrieben.

Ein weiteres großes Problem bei herkömmlichen Verfahren ist, die zu messenden intramolekularen Kräfte von unspezifischen Wechselwirkungen, insbesondere von den Wechselwirkungen zwischen der Messspitze und der zu vermessenden Oberfläche, zu unterscheiden. Dieses Problem kann durch flexible Linker reduziert, aber höchstens in Einzelfällen behoben werden. Experimente dieser Art sind in HINTERDORFER, P., BAUMGARTNER, W et al.: Detection and localization of individual antibodyantigen recognition events by atomic force microscopy, PNAS 93, 3477 (1996) und in ROS, R., SCHWESINGER, F. et al.: Antigen binding forces of individually addressed single-chain Fv antibody molecules, PNAS, 95, 7402 (1998) beschrieben.

Eine theoretische Erklärung für das Auftreten der beobachteten Haftkräfte auf der Grundlage der statistischen Mechanik wird für bestimmte Systeme in HANKE, F., LIVADARU, L., KREUZER, H. J.: Adsorption forces on a single polymer molecule in contact with a solid surface, Europhys. Lett., 69 (2), 242 (2005) gegeben.

Aus der DE 102 08 800 A1 ist ferner ein Verfahren zur Messung von Hafteigenschaften mit einem Rasterkraftmikroskop beschrieben. Dort wird eine Frequenzverschiebung in der dynamischen Rasterkraftmikroskopie zur Messung der Haftung verwendet. Dies ist eine indirekte Messung, da die Frequenz-Abstandskurven erst in Kraft-Abstandskurven konvertiert werden müssen. Außerdem konnte diese Methode nur im Ultrahochvakuum demonstriert werden.

Von besonderer Bedeutung ist in der Medizintechnik die Untersuchung der Beschichtung von Implantaten und auf dem Gebiet der Chemie die Untersuchung von Polymerbeschichtungen. Für beide Anwendungsfälle wurden Kraftmikroskope bislang nicht verwendet.

Zwar existieren heute eine Reihe von mechanischen Prüfverfahren für die Haftung von unterschiedlichen Beschichtungen. Der Anwendungsbereich ist aber insbesondere bei Werkstoffoberflächen aus Polymeren stark eingeschränkt und mit Nachteilen verbunden. Fast alle Prüfverfahren sind nicht zerstörungsfrei - zum Beispiel der Kratztest, Eindrücktest, Zugtest oder Vier-Punkt-Biegetest - oder auf organische Beschichtungen eingeschränkt anwendbar - zum Beispiel das Aufblasverfahren oder Abschälverfahren. In einigen Fällen, beispielsweise beim Zugtest oder beim Abschälverfahren, ist der Einsatz von Klebstoffen notwendig, wobei eine Reaktion der im Klebstoff vorhandenen Lösemittel mit der organischen Beschichtung nicht ausgeschlossen werden kann. Bedingt durch die Erzeugung von Spannungsfeldern, die mit dem Kontakt der Sonde mit der zu untersuchenden Beschichtungsoberfläche einhergeht, sind die mechanischen Haftuntersuchungen nur schwer unter gleichen Bedingungen und mit zufriedenstellender Wiederholungsrate durchzuführen. Durch die Erzeugung plastischer Verformungen von Substrat und Beschichtung durch den Messkopf der meisten Versuchsanordnungen kann sich ferner der experimentell zugängliche Wert für die Haftfestigkeit von dem tatsächlichen Wert oft um einige Größenordnungen unterscheiden. Quantitative Aussagen bezüglich der Haftung sind in allen Fällen nicht ohne große Unsicherheiten machbar.

Ausgehend von diesem Stand der Technik liegt der Erfindung daher die Aufgabe zugrunde, eine Vorrichtung und ein Verfahren anzugeben, mit denen sich zerstörungsfreie Messungen der Haftung von Beschichtungen und Verbundmaterialien unter verschiedenen Umgebungsbedingungen ausführen lassen.

Diese Aufgabe wird durch eine Vorrichtung und ein Verfahren mit den Merkmalen der unabhängigen Ansprüche gelöst. In davon abhängigen Ansprüchen sind vorteilhafte Ausgestaltungen und Weiterbildungen angegeben.

Bei der Vorrichtung wird an den Messkopf, der eine Relativbewegung bezüglich der Probenaufnahme ausführen kann, ein länglicher Träger angebracht, an den das Sondenelement angesetzt ist. Unter einem länglichen Träger soll in diesem Zusammenhang ein Träger verstanden werden, dessen Längeabmessung im gestreckten Zustand seine Querschnittsabmessungen übersteigt. Bei der Durchführung der Messung wird darüber hinaus ein der Haftung des Sondenelements an der Probe zuordenbares Kraftplateau erfasst. Dadurch kann die Haftung des Sondenelements an der Oberfläche der Probe mit großer Genauigkeit erfasst werden. Außerdem ist mit der Vorrichtung und dem Verfahren sichergestellt, dass das Sondenelement mit der Probe wechselwirkt, da die chemische Zusammensetzung des Trägers bekannt ist und die auf das Haften des Trägers an der Probe zurückgehenden Effekte eliminiert werden können.

Durch die Anordnung des Sondenelements abseits der beiden Enden des Trägers bildet sich oberhalb des dem Träger zugeordneten Kraftplateaus ein weiteres Kraftplateau aus, dessen Höhe und Länge bezüglich dem Kraftplateau, das dem Träger zuzuordnen ist, mit großer Genauigkeit bestimmt werden kann.

Bei einer bevorzugten Ausführungsform ist eine Vielzahl von Sondenelementen am Träger angebracht. Dadurch können bei einer einzelnen Messung mehrere nebeneinander angeordnete Kraftplateaus oder zumindest Kraftplateaus ausreichender Länge beobachtet werden und der Messfehler entsprechend reduziert werden.

Weiterhin wird für das Sondenelement vorzugsweise ein monomeres Sondenmolekül verwendet, da sich die Vorrichtung und das Verfahren insbesondere zur Untersuchung der Haftung von im Vergleich zu Polymeren kleinen, monomeren Sondenmolekülen, wie beispielsweise medizinischen Wirkstoffen oder Farbstoffen, eignet.

Für den Träger wird vorzugsweise ein Trägerpolymer verwendet, sofern es wenigsten eine Anbindungsmöglichkeit für das Sondenelement aufweist. Besonders geeignet erscheinen synthetisierte Spinnseide, Polypeptide, Polylactide und Polysaccharide oder andere Biopolymere.

Die Verbindung zwischen dem Messkopf und dem Träger wird vorzugsweise mithilfe von Polyethylenglycol bewerkstelligt. Dadurch kann die ungewollte unspezifische Adhäsion zwischen Messkopf und Probe sowie die Wechselwirkung des Sondenelements mit dem Messkopf unterdrückt werden.

Bei dem Verfahren für die Erfassung von Kräften im Submikronewton-Bereich kann die Länge des Kraftplateaus überwacht werden, um sicherzustellen, dass tatsächlich eine Wechselwirkung zwischen dem länglichen Träger und den Sondenelementen mit der Probe stattfindet.

Ferner kann die Differenz zwischen der Höhe des Kraftplateaus, das dem Träger zugeordnet ist, und dem Kraftplateau, das dem Sondenelement zugeordnet ist, ausgewertet werden, um die Haftung des Sondenelements an der Probe zu bestimmen. Insbesondere, wenn die Haftung des Trägers an der Probe vorab bekannt ist, kann das dem Träger zugeordnete Kraftplateau dazu verwendet werden, die Messung zu eichen und den Messfehler zu reduzieren.

Weitere Vorteile und Eigenschaften der Erfindung gehen aus der nachfolgenden Beschreibung hervor, in der Ausführungsbeispiele der Erfindung anhand der Zeichnung im Einzelnen erläutert werden. Es zeigen:
- Figur 1: die Darstellung eines Kraftmikroskops, dessen Messspitze mit einem länglichen Trägermolekül versehen ist, an das seitlich Sondenelemente angesetzt sind;
- Figur 2: ein typisches Plateau in einer Kraft-Abstandskurve für die Desorption eines Trägermoleküls von der Oberfläche einer Probe;
- Figur 3: ein Diagramm mit der Verteilung der Höhe der Kraftplateaus für die der Figur 2 zugrunde liegende Messung;
- Figur 4: ein Diagramm mit Kraft-Abstandskurven, die mit und ohne Sondenmoleküle aufgenommen worden sind;
- Figur 5: ein Diagramm mit der Verteilung der Niveauhöhe von Kraftplateaus bei einer Messung ohne Sondenmoleküle; und
- Figur 6: ein Diagramm mit der Verteilung der Niveauhöhe von Kraftniveaus der Messung mit Sondenmolekülen.

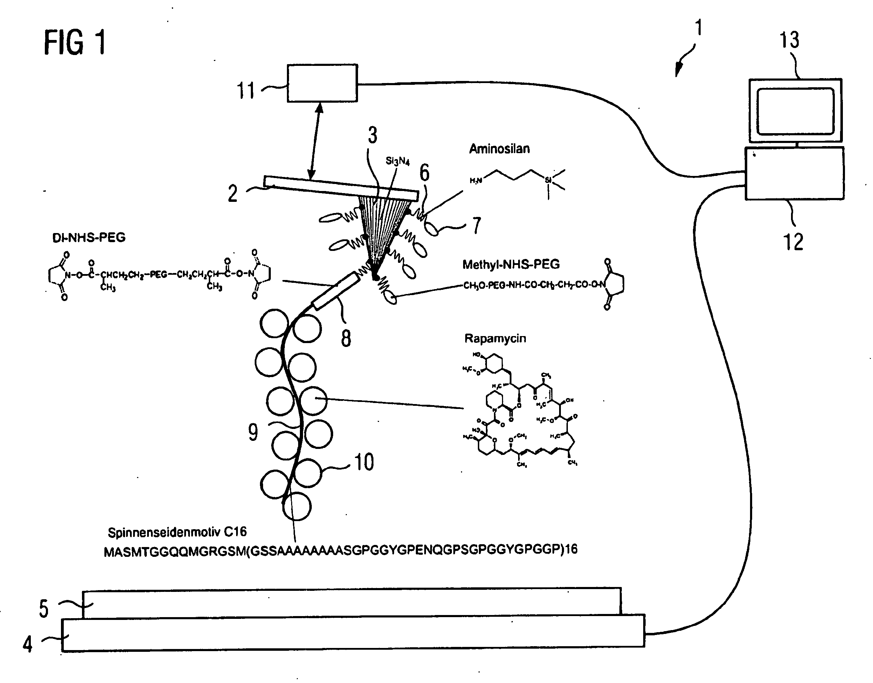

Figur 1 zeigt ein Kraftmikroskop 1, das eine an einem Ausleger 2 angebrachte Messspitze 3 aufweist. Das Kraftmikroskop 1 umfasst ferner einen Probenhalter 4, auf dem sich eine Probe 5 befindet.

Die Messspitze 3 ist mit Aktivierungsmolekülen 6 beschichtet, bei denen es sich beispielsweise um das in Figur 1 dargestellte Aminosilan handeln kann. Die freien Enden der Aktivierungsmoleküle sind mit Passivierungsmolekülen 7 und Verbindungsmolekülen 8 verbunden. Die Passivierungsmoleküle 7 können beispielsweise Methyl-NHS-PEG sein, deren freies Ende eine Methyl-Endgruppe aufweist. Die Verbindungsmoleküle 8 sind dagegen Di-NHS-PEG, die an beiden Enden eine Succinimidyl-Endgruppe aufweisen. An das Verbindungsmolekül 8 kann ein Trägermolekül 9 angesetzt werden, für das beispielsweise eine synthetisierte Spinnenseide, insbesondere das Spinnenseidenmotiv C16, verwendet werden kann. Seitlich an das Trägermolekül 9 sind Sondenmoleküle 10 angesetzt, die beispielsweise ein Wirkstoffmolekül, wie zum Beispiel Rapamycin, sein können. Wenn das Trägermolekül 9 oder die Sondenmoleküle 10 in Kontakt mit der Probe 5 gelangen, wird eine Haftkraft zwischen der Messspitze 3 und der Probe 5 vermittelt, die eine Bewegung der Messspitze 3 bewirkt. Die Bewegung der Messspitze 3 kann mithilfe eines Wegesensors 11 erfasst werden, dessen Messsignal eine Kontrolleinheit 12 beaufschlagt. Die Kontrolleinheit 12 kann beispielsweise ein handelsüblicher Arbeitsplatzrechner mit einem Bildschirm 13 sein. Die Kontrolleinheit 12 kann auch dazu dienen, den Probenhalter 4 zu verfahren.

Wie bereits erwähnt, kann als Trägermolekül 9 zum Beispiel eine synthetisierte Spinnenseide verwendet werden. Das in Figur 1 als Trägermolekül 9 verwendete Polymer, nämlich Spinnenseidenmotiv C16, hat 16 Carboxylgruppen, an die ein Wirkstoff über seine Hydroxylgruppen mittels einer selektiven Veresterung angebunden werden kann.

Neben der synthetisierten Spinnenseide kommen weitere Stoffe und Stoffgruppen als Trägermolekül 9 infrage, sofern diese die Möglichkeit der Anbindung an die Messspitze 3 und die Möglichkeit der Anbindung der Sondenmoleküle 10 bieten.

Beispielsweise kommen Polypeptide als Trägermoleküle 9 infrage, sofern diese es gestatten, die Sondenmoleküle 10 anzubinden. Ein Beispiel für diese Stoffgruppe stellt die poly(Glutaminsäure) dar, bei der der die gleiche Anbindungschemie wie bei C16 möglich ist. Auch Polylactide eignen sich für das Trägermolekül 9, da diese einerseits als biokompatibles Polymer in der Beschichtung von Medizinprodukten häufig zum Einsatz kommen und andererseits in Verbindung mit Wirkstoffen kommerziell erhältlich sind. Ein Vertreter dieser Stoffgruppe stellt beispielsweise der Komplex von Poly(D,Llactid) und Rapamycin dar, der in der interventionellen Kardiologie als Stentbeschichtung zum Einsatz kommt.

Die Messspitze 3 kann beispielsweise präpariert werden, indem die als Haftsonde eingesetzte Messspitze 3 des Kraftmikroskops 1 entweder direkt, nach Aufbringen einer Oxid- oder Metallschicht oder nach der Oxidation im Plasma oder Säurebad unter Zuhilfenahme eines Silans, Thiols oder Disulfids mit einer Monolage aus Polyethylenglycol (= PEG) einer oder unterschiedlicher Funktionalität beschichtet wird. Die Ankopplung des Trägermoleküls 9 erfolgt dann über die kovalente Anbindung an PEG.

Im Einzelnen kann die Messspitze 3 wie folgt präpariert werden: Eine Messspitze 3 aus Siliziumnitrid wird nach einer nasschemischen Reinigung in einer Lösung aus konzentrierter Schwefelsäure und Kaliumdichromat und nach einer weiteren Feinreinigung und Aktivierung im Sauerstoffplasma mit einem Aminosilan beschichtet. Diese modifizierte Messspitze wird mit einer weiteren Schicht aus einem Gemisch aus Methoxy-NHS-PEG (Methyl-Endgruppe) und einem bifunktionalen Di-NHS-PEG (Succinimidyl-Endgruppen) versehen. Die Molekulargewichte und Mischungsverhältnisse werden so angepasst, dass ein einzelnes Trägermolekül 9 vermessen werden kann. Die Beimischung von Methoxy-PEG unterdrückt die ungewollte unspezifische Adhäsion zwischen Messspitze 3 und Probe 5 sowie die Wechselwirkung des Trägermoleküls 9 mit der Messspitze 3. Im Anschluss wird der N-Terminus des synthetischen Spinnenseidenmotivs C16 kovalent an die noch freien Bindungsstellen des Di-NHS-PEGs angebunden.

Mit dem in Figur 1 dargestellten Kraftmikroskops 1 können Messverfahren ausgeführt werden, mit denen die Haftung auf molekularer Ebene erfasst werden kann. Dazu wird das Kraftmikroskop 1 mit einem einzelnen Trägermolekül 9 verwendet.

Das einzelne Trägermolekül 9 wird spezifisch an die Messspitze 3 angebunden, in Kontakt mit der zu untersuchenden Oberfläche gebracht und die beim Zurückziehen des Trägermoleküls 9 nötige Kraft gemessen. Unter geeigneten Bedingungen, insbesondere in wässriger Umgebung, können Kraftplateaus in der Kraft-Abstandskurve beobachtet werden. Die Höhe dieser Kraftplateaus ist äußerst empfindlich auf die Haftkraft zwischen Trägermolekül 9 und Probe 5 und unabhängig von der Kraftladungsrate. Unter Kraftladungsrate wird in diesem Zusammenhang die Steigung der Kraft-Zeit-Kurve während des Zurückziehvorgangs verstanden. Typischerweise liegen die Haftkräfte im Bereich von 10 bis einigen 100 Pikonewton. Mit dem Kraftmikroskop 1 können daher sehr feine Unterschiede in der Haftung von Beschichtungen gemessen werden.

Es sei angemerkt, dass der Verlauf der Kraftkurve auf der Zeitskala des Zurückziehvorgangs nicht von der Zuggeschwindigkeit abhängig ist. Denn bei den verwendeten Zuggeschwindigkeiten kleiner als 1 mm/s werden Quasi-Gleichgewichtskräfte gemessen.

Die Länge der Kraftplateaus ist im Wesentlichen gleich der Länge der Trägermoleküle 9. Die Fläche der Kraftplateaus ist ferner im Wesentlichen gleich der beim Zurückziehen des Trägermoleküls 9 geleisteten Arbeit, die aufgebracht werden muss, um das Trägermolekül 9 aus dem Oberflächenpotentialtopf heraus zu befördern. Die beim Zurückziehen aufgewandte Energie für das Strecken des Trägermoleküls 9 wird dabei allerdings nicht berücksichtigt.

Der gleichmäßige Verlauf des Kraftplateaus wird anschaulich verständlich, wenn man sich vor Augen hält, dass bei schrittweisem Zurückziehen des Trägermoleküls 9 die geleistete Arbeit gleich derjenigen Arbeit ist, die aufgewendet werden muss, um einen Abschnitt des Trägermoleküls 9 vom freien Ende des Trägermoleküls 9 zur Messspitze 3 zu befördern.

Insbesondere in der Medizintechnik ist oft eine gezielte und zeitlich steuerbare Freisetzung von medizinischen Wirkstoffen von einer Oberfläche aus notwendig. Mit dem Krafmikroskop 1 ist es möglich, die Haftung medizinischer Wirkstoffe an unterschiedlichen Stellen sehr schnell zu erfassen und damit die Oberfläche mit dem optimalen Freisetzungsverhalten zu finden. Außerdem lässt sich mit der hier beschriebenen Vorrichtung die Haftung von zu Oberflächenbeschichtung verwendeten Stoffen, beispielsweise von Farbstoffen, untersuchen.

Das Kraftmikroskop 1 ist für diesen Anwendungszweck besonders geeigent, da sich mit der hier beschriebenen Vorrichtung und dem hier beschriebenen Verfahren die Haftung von im Vergleich zu Polymeren kleinen, monomeren Molekülen untersuchen lassen. In diese Klasse fallen die meisten in der Medizin eingesetzten Wirkstoffe. Aber auch gebräuchliche Farbstoffe lassen sich dieser Klasse zuordnen. Werden die Sondenmoleküle 10 direkt an die Messspitze 3 angebunden, kann kein Plateau in der Kraft-Abstandskurve beobachtet werden. Daher wird hier ein langes Molekül als Trägermolekül 9 verwendet, an welches ein einzelnes oder eine Vielzahl der im Vergleich zum Trägermolekül kleinen Sondenmoleküle 10, deren Haftung untersucht werden soll, angebunden wird. Insbesondere bei Messungen mit kleinen Molekülen ist es von Vorteil, wenn wenigstens zwei der Sondenmoleküle 10 an dem Trägermolekül 9 angebracht sind. Bei ausreichender Größe der Sondenmoleküle 10 kann gelegentlich auch ein einzelnes Sondenmolekül 10 ausreichend sein.

Im Folgenden werden verschiedene Messungen, die mit einem gemäß Figur 1 präparierten Messspitze 3 vorgenommen worden sind, näher erläutert.

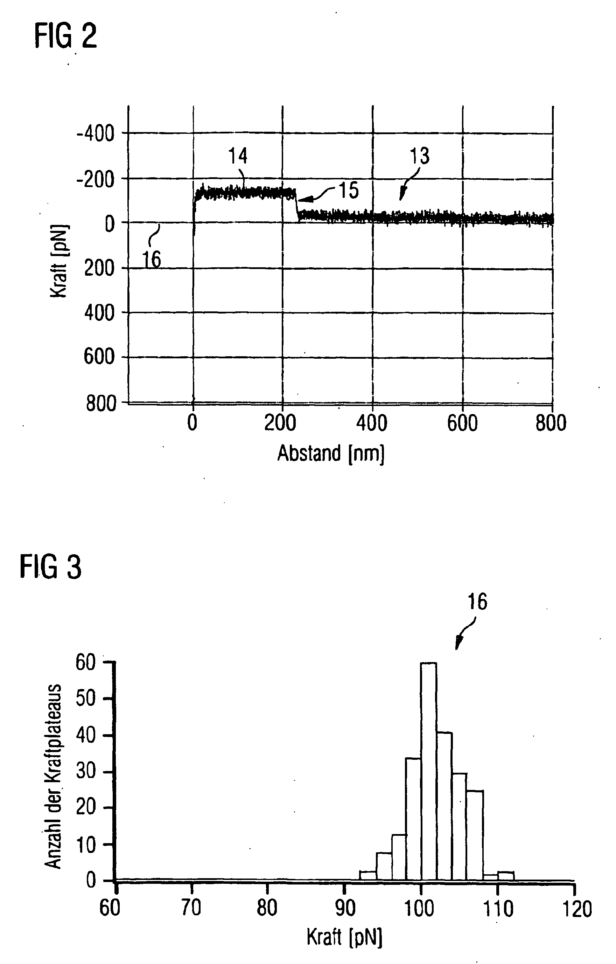

In den Figuren 2 und 3 sind Ergebnisse von Messungen dargestellt, die mit einem einzelnen polymeren Trägermolekül 9 vorgenommen worden sind. Insbesondere wurde die Haftstärke des Spinnenseidemoleküls, insbesondere des Spinnenseidenmotivs C16 mit einer definierten Länge von 230 nm, auf einer mit Wasserstoff terminierten Diamantoberfläche in wässriger Lösung untersucht. Es wurden auf einer typischen Zeitskala (hier 1 µm/sec) Kraftabstandskurven an verschiedenen Stellen der Unterlage aufgenommen und die gemessenen Kraftplateaus bezüglich Länge und Desorptionskraft ausgewertet. In Figur 2 ist beispielsweise eine Kraft-Abstands-Kurve 13 mit einem Kraftplateau 14 dargestellt. Hierbei indiziert ein Abfall 15 auf eine Nulllinie 16 das Ablösen des Trägermoleküls 9 von der Oberfläche der Probe 5.

Es sei angemerkt, dass die Kraft-Abstandskurve 13 im Wesentlichen durch das Kraftplateau 14 geprägt ist. Dieser Umstand beruht auf einer Optimierung der verwendeten Messspitze 3, die eine Wechselwirkung zwischen der Messspitze 3 und dem Trägermolekül 9 ausschließt.

Figur 3 zeigt eine Verteilung der Plateauhöhen für 220 Plateaus für das Experiment in Figur 1.

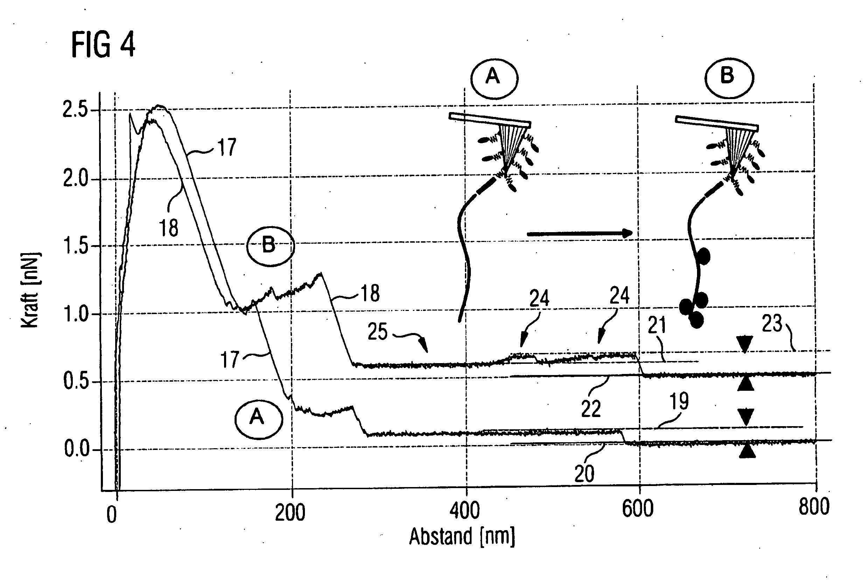

In den Figuren 4 bis 6 sind weiterhin die Ergebnisse eines Experiment gezeigt, bei dem zuerst die Haftung eines Trägermoleküls 9, nämlich Polyallylamine, auf einer mit Wasserstoff terminierten Diamant-Oberfläche gemessen wird (A) und dann die Haftung desselben Trägermoleküls 9 mit Farbstoffmolekülen (B) an der gleichen Oberfläche bestimmt wurde. Bei den Farbstoffmolekülen handelt es sich um Moleküle des Farbstoffs Promofluor 555 der Firme PromoKine.

Die Messungen wurden in PBS-Puffer bei etwa 37 °C durchgeführt, um die Eignung für medizinisch relevante Moleküle zu unterstreichen.

Figur 4 zeigt zu Vergleichszwecken zwei Desorptionskurven 17 und 18 von Polyallylamin von H-Diamant bei 36.5 °C in PBS ohne (A) und mit (B) Promofluor 555-Molekülen.

Bei der Desorptionskurve 17 ergibt sich die Plateaukraft aus der Differenz einer Plateaulinie 19 mit einer Nulllinie 20.

Bei der Desorptionskurve 18 kann die für das Trägermolekül 9 geltende Plateaukraft zunächst aus der Differenz von Plateaulinie 21 und Nulllinie 22 ermittelt werden. Die Anbindung von stärker haftenden kleineren Sondenmolekülen 10 führt zu einer Verschiebung der Plateaulinie zu der Plateaulinie 23. Die erhöhte Haftkraft F_{Des} kann demnach durch eine Erhöhung der Plateaukraft gemessen werden.

Das Verschieben der Plateaulinie 23 wird durch das Auftreten zusätzlicher Kraftplateaus 24 hervorgerufen, die oberhalb des Kraftplateaus 25 liegen, das der Desorption der Trägermoleküls 9 von der Oberfläche der Probe 5 zuzuordnen ist. Die zusätzlichen Kraftplateaus 24 beruhen auf der Desorption der Sondenmoleküle 10 von der Oberfläche der Probe 5.

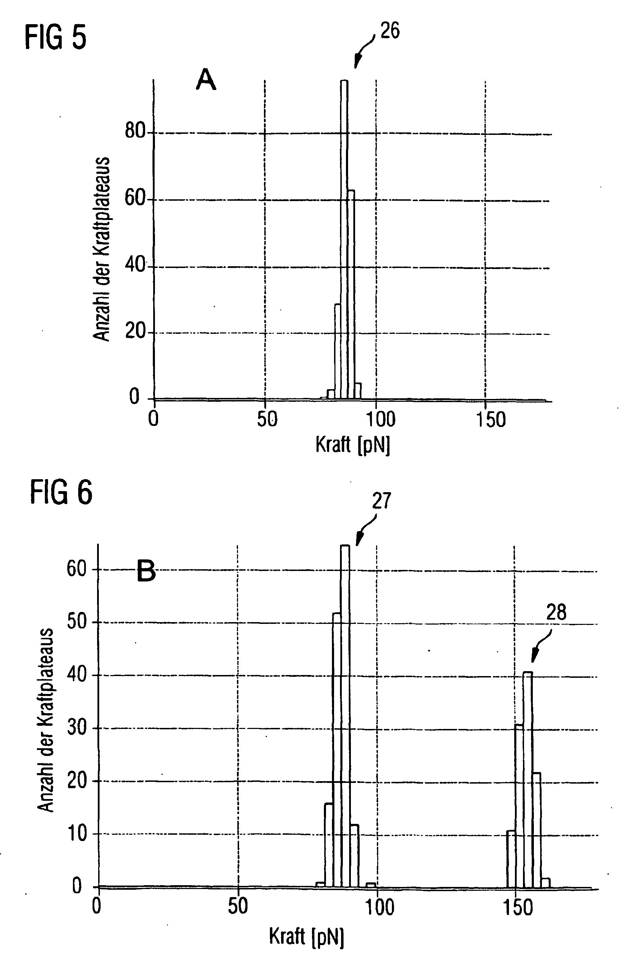

Figur 5 und 6 zeigen zu Vergleichszwecken die jeweilige Kraftverteilung von Polyallylamin ohne (A) und mit (B) Promofluor 555. Während die in Figur 5 dargestellte Kraftverteilung für den Fall (A) eine einzelne Spitze 26 zeigt, treten bei der in Figur 6 dargestellten Kraftverteilung für den Fall (B) zwei Spitzen 27 und 28 auf.

Der Mittelwert der in Figur 5 dargestellten Spitze 26 liegt bei etwa 86 Piconewton. Die Haftkraft des reinen Trägerpolymers ist daher etwa 86 Piconewton. Dies entspricht einer Desorptionsenergie von etwa 7 k_{B}T (= 3x10⁻²⁰ Joule) pro Aminosäure.

Figur zeigt die Kraftverteilung für den Fall, bei dem die reaktive Farbstoffe (Promofluor 555, Mw = 793 Dalton, PromoKine) an genau dieses Trägerpolymer angebunden und wieder die Plateaukräfte bestimmt worden sind. Gemäß Figur 4 entstehen zusätzlich höhere Kraftplateaus 24 und damit neben der auf die Desorption des Trägermoleküls 9 zurückgehende ersten Spitze 27 eine zweite Spitze 28 der in Figur 6 dargestellten Verteilung der Plateauhöhen mit einer Haftkraft von etwa 153 Piconewton. Dies entspricht einer Desorptionsenergie von mehr als 12 k_{B}T (5x10⁻²⁰ Joule) pro Aminosäure.

Es sei angemerkt, dass die der Plateaulinie 21 entsprechende Plateauhöhe des reinen Trägerpolymers in Figur 4 noch deutlich zu sehen ist, da es nicht vollständig mit Fluorophoren belegt ist. Die Haftkraft und damit Desorptionsenergie hat sich jedoch deutlich geändert.

Dieses Experiment zeigt, wie sich die Haftkraft eines einzelnen Trägerpolymers durch die Anbindung von kleinen Sondenmolekülen 10, insbesondere Farbstoffen, an genau dieses Trägerpolymer verändert. In diesem Fall erhöht sich die Haftkraft auf der hydrophoben Oberfläche wie erwartet, da der Farbstoff hydrophober als das Trägerpolymer ist. Dies bestätigt, dass tatsächlich in einer Gleichgewichtsmessung die Haftung der Sondenmoleküle 10 und nicht nur die Haftung des Trägerpolymers gemessen wurde.

Es sei angemerkt, dass als Trägermolekül 9 vorzugsweise ein Polymer mit bekannter Länge verwendet wird, wodurch eine sichere intrinsische Kontrolle gegeben ist, dass auch wirklich die zu untersuchende Haftung zwischen Sondenmolekül 10 und Oberfläche gemessen wird.

Ferner sei angemerkt, dass das auf die Desorption des Trägermoleküls 9 zurückgehende Kraftplateau 25 dazu verwendet werden kann, die Desorptionskurve 18 zu eichen, da die für die Desorption des Trägermoleküls 9 notwendige Desorptionsenergie in der Regel aus vorherigen Messungen bekannt ist. Dadurch kann der Fehler bei der Bestimmung der für die Desorption der Sondenmoleküle 10 notwendigen Desorptionsenergie beträchtlich reduziert werden.

Aufgrund der kleineren Fehlers kann die Vorrichtung auch zur Messung des sehr kurzreichweitigen (< 1 nm) Regimes der hydrophoben Kraft verwendet werden. Dieses konnte bisher mit noch keiner Methode gemessen werden und es gibt Hinweise, beispielsweise in MEYER, E.E., ROSENBERG, K.J., ISRAELACHVILI, J.: Recent progress in understanding hydrophobic interactions, PNAS, 103, 15739 (2006), dass die Kraft in diesem Regime viel größer als in den bisher gemessenen mittel- und langreichweitigen Regimes der hydrophoben Kraft sind.

Dass mit dem Kraftmikroskop 1 diese Kräfte tatsächlich gemessen werden können, zeigen erste Messungen auf verschiedenen Oberflächen mit unterschiedlichen Ionen und Pufferstärken. Dabei wurden auf hydrophoben Oberflächen Kraftunterschiede beobachtet, die nicht durch die Elektrostatik oder andere langreichweitige Kräfte zu erklären sind.

Die Vorrichtung und das Verfahren können auf verschieden Art und Weise abgewandelt werden.

Statt eines Kraftmikroskopes können auch andere Vorrichtungen zur Messung von Kräften im Submikronewton-Bereich verwendet werden. Derartige Vorrichtungen sind zum Beispiel Nanoindenter, optische Pinzetten, magnetische Pinzetten oder andere Vorrichtungen zum Erfassen von Oberflächenkräften.

Statt eines einzelnen Polymers können auch mehrere Polymere als Trägermoleküle verwendet werden, sofern es ein Kraftplateau in der Kraft-Abstandskurve gibt, wie es zum Beispiel bei Dreiersträngen aus poly(Glycin-Valin-Glycin-Valin-Prolin) zu erwarten ist.

Anstelle einer Veresterung können auch eine andere kovalente Anbindungen für die Anbindung des Sondenmoleküls an das Trägermolekül verwendet werden. Insbesondere sei hier die so genannte Click-Chemie genannt. Nähere Angabe zur Click-Chemie finden sich beispielsweise in KOLB, H.C., FINN, M.G., SHARPLESS, K.B., Click Chemistry: Diverse Chemical Function from a Few Good Reactions, Angewandte Chemie Int. Ed. Vol. 40, 2004 (2001).

Ferner können statt eines Plateaus in der Kraft-Abstandskurve auch andere charakteristische Merkmale in der Kraft-Abstands-kurve herangezogen werden, zum Beispiel der Sessel-Wanneübergang bei Polysacchariden oder Abfolgen von Kraftplateaus mit verschiedenen Höhen.

Weiterhin ist es möglich eine Vielzahl von Messungen parallel durchzuführen. Denn statt einer Messspitze können gleichzeitig mehrere Messspitzen verwendet werden.

Es sei hervorgehoben, dass die Messspitze vorzugsweise so zu modifizieren ist, dass das Trägermolekül keine Wechselwirkung mit der Messspitze hat.

Ferner sei angemerkt, dass für eine erste grobe Charakterisierung auch die drei folgenden diskreten Formen der Kraft-Abstandskurve beim Zurückziehen der Messspitze hilfreich sind: beispielsweise kann der Verlauf des Anfangsbergs in der Desorptionskurve 18 ausgewertet werden oder zusätzliche Informationen aus dem schanzenartigen Verlauf der Desorptionskurve 18 vor dem Abbruch bei einem Abstand von 250 nm gezogen werden.

Die Nutzung des Sondenmoleküls als Trägermolekül für kleinere nichtpolymere Moleküle ermöglicht aufgrund der Detektion von Haftungskräften den Einsatz der hier beschriebenen Verfahren im Bereich des Wirkstoffscreenings und insbesondere durch die Möglichkeit eine Vielzahl von Sondenmolekülen 10 an das Trägermolekül 9 anzukoppeln auch für das Massenscreening von Wirkstoffen. Die Wirkstoffbeladene Messspitze 3 wird hierbei auf einer geeigneten Unterlage mit immobilisierten Rezeptoren eingesetzt. Die Beschleunigung bei der Detektion von Wechselwirkungskräften durch das vorliegende Verfahren bedingt durch die Messung im Gleichgewicht ist hier als großer Vorteil gegenüber bestehenden Verfahren anzuführen. Auch der umgekehrte Fall einer mit Rezeptormolekülen beladene Sonde, die von einer Wirkstoffunterlage desorbiert wird, ist möglich.

Eine weitere Anwendungsmöglichkeit im Bereich der Biotechnologie und Pharmazie besteht in der Verwendung von organischen Säuren in polymerer Form, wie zum Beispiel DNA oder RNA als Sondenmoleküle. Die Analyse der Kraftplateaus gibt Aufschluss über die Nukleinsäuresequenz entlang der Polymerkette. Die Sequenzierung von DNA- oder RNA-Strängen kann auch dadurch erfolgen, dass zunächst die zu testenden DNA- oder RNA-Moleküle auf einer Probenaufnahme immobilisiert, die komplementäre Basen an ein Trägerpolymer angekoppelt werden und deren Haftung gemäß dem hier beschriebenen Verfahren gemessen wird.

Die hier beschriebene Vorrichtungen und die hier beschriebene Verfahren weisen eine Reihe von weiteren Vorteilen auf:

Zunächst ist hervorzuheben, dass das Kraftmikroskope bei vielen Umgebungen, zum Beispiel physiologischen Bedingungen, Raumtemperatur oder atmosphärischem Druck, funktioniert.

Wie oben beschrieben hängt die gemessene Bindungskraft von der Kraftladungsrate ab. Diese Abhängigkeit wurde bei herkömmlichen Verfahren, insbesondere bei den in der EP 0 727 639 A1 und der EP 0 829 722 A2 bekannten Verfahren nicht berücksichtigt. Infolgedessen war es notwendig, hunderte von Abrisskurven bei unterschiedlichen Kraftladungsraten aufzunehmen und diese miteinander zu korrelieren, um einen aussagekräftigen quantitativen Wert für die Haftung zu erhalten. Wie die in Figur 3 dargestellte enge Kraftverteilung innerhalb eines Experimentes zeigt, genügt bei der hier beschriebenen Vorrichtung und dem hier beschriebenen Verfahren im Prinzip eine einzige Kraftkurve, um die genaue Haftkraft zu messen.

Wie bereits eingangs erwähnt wurde, stellt es ein großes Problem bei herkömmlichen Verfahren dar, die zu messenden intramolekularen Kräfte von unspezifischen Wechselwirkungen, insbesondere von den Wechselwirkungen zwischen der Messspitze und der zu vermessenden Oberfläche, zu unterscheiden. Dieses Problem kann durch flexible Linker reduziert aber höchstens in Einzelfällen behoben werden. Demgegenüber ist die definierte Länge des als Trägermoleküls eingesetzten Polymers, die mit der Länge des Kraftplateaus übereinstimmen muss, ein sicherer Indikator dafür, dass die gemessene Kraft tatsächlich zwischen dem Sondenmolekül und der Oberfläche wirkt.

Ein weiteres Problem herkömmlicher Verfahren ist, dass die Oberfläche und Beschichtung der Messspitze auf molekularer Ebene nie genau gleich ist. Vielmehr ist die Beschichtung auf molekularer Ebene inhomogen. Damit werden mit zwei identisch präparierten Messspitzen auf derselben Oberfläche unterschiedlich hohe Abrisskräfte gemessen. Bei dem hier beschriebenen Verfahren liefert dagegen jede Messspitze bei der Vermessung eines Kraftplateaus das gleiche Ergebnis, denn ein einzelnes Polymer ist zu 100% rein. Ferner konnte außerdem die Stabilität der Sonde über viele hundert Kraftkurven gezeigt werden. Auf diese Weise macht es das hier beschrieben Verfahren möglich, viele Unterlagen mit ein und demselben Molekül zu vermessen und dadurch eine optimale Vergleichbarkeit und Reproduzierbarkeit der Ergebnisse zu generieren.

Mit den Verfahren des Standes der Technik kann die Haftung von Beschichtungen nur mit großen Einschränkungen gemessen werden. Vielmehr handelt sich bei den bekannten Messverfahren um Verfahren, die grobe Abschätzungen liefern. Insofern erscheint ein großtechnischer Einsatz der bekannten Verfahren wenig sinnvoll. Demgegenüber kann mit dem hier beschriebenen Verfahren nicht nur die Haftung von Polymeren, sondern auch von Molekülen, wie zum Beispiel vieler handelsüblicher medizinischer Wirkstoffe oder Farbstoffe, bestimmt werden.

Abschließend sei noch darauf hingewiesen, dass Merkmale und Eigenschaften, die im Zusammenhang mit einem bestimmten Ausführungsbeispiel beschrieben worden sind, auch mit einem anderen Ausführungsbeispiel kombiniert werden können, außer wenn dies aus Gründen der Kompatibilität ausgeschlossen ist.

Schließlich wird noch darauf hingewiesen, dass in den Ansprüchen und in der Beschreibung der Singular den Plural einschließt, außer wenn sich aus dem Zusammenhang etwas anderes ergibt. Insbesondere wenn der unbestimmte Artikel verwendet wird, ist sowohl der Singular als auch der Plural gemeint.

## Patentansprüche

1. Vorrichtung für die Erfassung von Kräften im Submikronewton-Bereich mit:
- einem Messkopf (3), mit dem eine Relativbewegung bezüglich einer Probenaufnahme (4) ausführbar ist,
- wenigstens einem an den Messkopf (3) angebrachten Sondenelement (10), durch das eine Haftkraft zwischen einer auf der Probenaufnahme (4) angeordneten Probe (5) und dem Messkopf (3) vermittelbar ist,
- einem an dem Messkopf (3) angebrachten länglichem Träger (9), an den das Sondenelement (10) angesetzt ist, und mit
- einer Auswerteeinheit (12), mit der die Relativbewegung zwischen Messkopf (3) und Probenaufnahme (4) detektierbar ist, und durch die ein der Haftung des Sondenelements (10) an der Probe zugeordnetes Kraftplateau (24) erfassbar ist, **dadurch gekennzeichnet, dass**
- das Sondenelement (10) an den Träger (9) abseits der beiden Enden des Trägers (9) angesetzt ist und dass
- an den länglichen Träger (9) wenigstens zwei Sondenelemente (10) angesetzt sind.

2. Vorrichtung nach Anspruch 1,
**dadurch gekennzeichnet, dass** an den länglichen Träger (9) eine Vielzahl von Sondenelementen (10) angesetzt ist.

3. Vorrichtung nach Anspruch 1 oder 2,
**dadurch gekennzeichnet, dass** das Sondenelement (10) ein monomeres Molekül ist.

4. Vorrichtung nach Anspruch 3,
**dadurch gekennzeichnet, dass** das Sondenelement (10) ein medizinischer Wirkstoff oder ein Farbstoff ist.

5. Vorrichtung nach einem der Ansprüche 1 bis 4,
**dadurch gekennzeichnet, dass** der Träger ein Trägerpolymer (9) mit Anbindungsmöglichkeiten für das Sondenelement (10) ist.

6. Vorrichtung nach Anspruch 5,
**dadurch gekennzeichnet, dass** das Trägerpolymer (9) eine vorbestimmte Länge aufweist.

7. Vorrichtung nach Anspruch 5 oder 6,
**dadurch gekennzeichnet, dass** das Trägerpolymer (9) auf der Grundlage eines Biopolymers hergestellt ist.

8. Vorrichtung nach Anspruch 7,
**dadurch gekennzeichnet, dass** das Trägerpolymer (9) auf der Grundlage einer synthetisierten Spinnseide, eines Polypeptids, eines Polylactids oder Polysaccherids hergestellt ist.

9. Vorrichtung nach einem der Ansprüche 1 bis 8,
**dadurch gekennzeichnet, dass** der Messkopf (3) mit Polyethylenglycol beschichtet ist.

10. Verfahren für die Erfassung von Kräften im Submikronewton-Bereich, bei dem der Abstand zwischen einem mit einem Sondenelement (10) versehenen Messkopf (3) und einer Probe (5) variiert wird und ein Zusammenhang zwischen einer zwischen dem Messkopf (3) und der Probe (5) wirkenden Haftkraft mit Hilfe eines Sondenelements (10) ermittelt wird, das an einen am Messkopf (3) angebrachten länglichen Träger (9) angesetzt ist,
**dadurch gekennzeichnet, dass**
- das Sondenelement (10) an den Träger (9) abseits der beiden Enden des Trägers (9) angesetzt ist und dass
- ein der Haftung des Sondenelements (10) an der Probe zuordenbares Kraftplateau (24) erfasst wird.

11. Verfahren nach Anspruch 10,
**dadurch gekennzeichnet, dass** als Sondenelement ein monomeres Sondenmolekül (10) verwendet wird.

12. Verfahren nach einem der Ansprüche 10 oder 11,
**dadurch gekennzeichnet, dass** als Träger ein Trägerpolymer (9) mit Anbindungsmöglichkeit für das Sondenelement (10) verwendet wird.

13. Verfahren nach einem der Ansprüche 10 bis 12,
**dadurch gekennzeichnet, dass** ein Träger (9) vorbestimmter Länge verwendet wird.

14. Verfahren nach einem der Ansprüche 10 bis 13,
**dadurch gekennzeichnet, dass** die Länge eines Kraftplateaus (25), das dem Träger (9) zugeordnet ist, überwacht wird.

15. Verfahren nach einem der Ansprüche 10 bis 14,
**dadurch gekennzeichnet, dass** die Differenz der Höhe eines Kraftplateaus (24) des Sondenelements (10) zur Höhe des Kraftplateaus (25) des Trägers (9) ausgewertet wird.

## Claims

1. An apparatus for the detection of forces in the sub-micronewton range, with:
- a measuring head (3) with which a relative movement in relation to a sample holder (4) can be carried out,
- at least one probe element (10) attached to the measuring head (3), by which probe element an adhesion force between a sample (5) placed on the sample holder (4) and the measuring head (3) can be transferred,
- an elongate carrier (9) attached to the measuring head (3), on which elongate carrier (9) the probe element (10) is placed, and with:
- an evaluation unit (12) with which the relative movement between said measuring head (3) and said sample holder (4) can be detected and by which a force plateau (24) associated to the adhesion of the probe element (10) to the sample can be detected,
**characterized in that**
- the probe element (10) is placed on the carrier (9) remote from both ends of the carrier (9), and that
- at least two probe elements (10) are placed on the elongate carrier (9).

2. The apparatus according to claim 1,
**characterized in that**
a multiplicity of probe elements (10) are placed on the elongate carrier (9).

3. The apparatus according to anyone of the claims 1 or 2,
**characterized in that**
the probe element (10) is a monomeric molecule.

4. The apparatus according to claim 3,
**characterized in that**
the probe element (10) is a medical agent or a dye.

5. The apparatus according to anyone of the claims 1 to 4,
**characterized in that**
the carrier is a carrier polymer (9) with binding possibilities for the probe element (10).

6. The apparatus according to claim 5,
**characterized in that**
the carrier polymer (9) has a predetermined length.

7. The apparatus according to claim 5 or 6,
**characterized in that**
the carrier polymer (9) is made on the basis of a biopolymer.

8. The apparatus according to claim 7,
**characterized in that**
the carrier polymer (9) is made on the basis of a manmade spider silk, a polypeptide, a polylactide, or a polysaccharide.

9. The apparatus according to anyone of the claims 1 to 8,
**characterized in that**
the measuring head (3) is coated with polyethylene glycol.

10. A method for the detection of forces in the sub-micronewton range, wherein the distance between a measuring head (3) provided with a probe element (10) and a sample (5) is varied and a relation to an adhesion force acting between the measuring head (3) and the sample (5) is determined with the aid of a probe element (10) which is placed on an elongate carrier (9) attached to the measuring head (3),
**characterized in that**
a probe element (10) is attached to the elongate carrier (9) remote from both ends of the carrier (9), and
a force plateau (24) to be associated with the adhesion of the probe element (10) to the sample is detected.

11. The method according to claim 10,
**characterized in that**
a monomeric probe molecule (10) is used as a probe element.

12. The method according to anyone of the claims 10 or 11,
**characterized in that**
as a carrier a carrier polymer (9) with a binding possibility for the probe element (10) is used.

13. The method according to anyone of the claims 10 to 12,
**characterized in that**
a carrier (9) having a predetermined length is used.

14. The method according to anyone of the claims 10 to 13,
**characterized in that**
the length of a force plateau (25) associated with the carrier (9) is surveyed.

15. The method according to anyone of the claims 10 to 14,
**characterized in that**
the difference of the level of a force plateau (24) of the probe element (10) to the level of the force plateau (25) of the carrier (9) is evaluated.

## Revendications

1. Dispositif pour enregistrer des forces inférieures au micronewton, comportant :
- une tête de mesure (3), à l'aide de laquelle peut être réalisé un mouvement relatif par rapport à un porte-échantillons (4),
- au moins un élément formant sonde (10), rapporté à la tête de mesure (3), grâce auquel il est possible de fournir une force d'adhérence entre un échantillon (5) disposé sur le porte-échantillons (4) et la tête de mesure (3),
- un support oblong (9), rapporté à la tête de mesure (3), auquel est fixé l'élément formant sonde (10), et comportant
- une unité d'évaluation (12), à l'aide de laquelle le mouvement relatif entre la tête de mesure (3) et le porte-échantillons (4) peut être détecté, et grâce à laquelle l'adhérence de l'élément formant sonde (10) au plateau de force (24) affecté à l'échantillon peut être enregistrée,
**caractérisé en ce que**
- l'élément formant sonde (10) est rapporté au support (9) à l'écart des deux extrémités du support (9), et **en ce que**
- au moins deux éléments formant sonde (10) sont rapportés au support oblong (9).

2. Dispositif selon la revendication 1, **caractérisé en ce qu'**un grand nombre d'éléments formant sonde (10) sont rapportés au support oblong (9).

3. Dispositif selon la revendication 1 ou 2, **caractérisé en ce que** l'élément formant sonde (10) est une molécule monomère.

4. Dispositif selon la revendication 3, **caractérisé en ce que** l'élément formant sonde (10) est un principe actif médical ou un colorant.

5. Dispositif selon l'une des revendications 1 à 4, **caractérisé en ce que** le support est un polymère (9) formant support, avec des possibilités de liaison pour l'élément formant sonde (10).

6. Dispositif selon la revendication 5, **caractérisé en ce que** le polymère formant support (9) présente une longueur prédéfinie.

7. Dispositif selon la revendication 5 ou 6, **caractérisé en ce que** le polymère formant support (9) est fabriqué sur la base d'un biopolymère.

8. Dispositif selon la revendication 7, **caractérisé en ce que** le polymère formant support (9) est fabriqué sur la base d'une soie de filature synthétisée, d'un polypeptide, d'un polylactide ou d'un polysaccharide.

9. Dispositif selon l'une des revendications 1 à 8, **caractérisé en ce que** la tête de mesure (3) est revêtue de polyéthylèneglycol.

10. Procédé pour l'enregistrement de forces inférieures au micronewton, dans lequel on fait varier la distance entre une tête de mesure (3) pourvue d'un élément formant sonde (10) et un échantillon (5), et on détermine une relation entre une force d'adhérence agissant entre la tête de mesure (3) et l'échantillon (5) à l'aide d'un élément formant sonde (10) qui est fixé à un support oblong (9) rapporté à la tête de mesure (3),
**caractérisé en ce que**
- l'élément formant sonde (10) est fixé au support (9) à l'écart des deux extrémités du support (9), et **en ce que**
- un plateau de force (24), pouvant être affecté à l'adhérence de l'élément formant sonde (10) à l'échantillon, est enregistré.

11. Procédé selon la revendication 10, **caractérisé en ce qu'**on utilise en tant qu'élément formant sonde une molécule sonde monomère (10).

12. Procédé selon l'une des revendications 10 ou 11, **caractérisé en ce qu'**on utilise en tant que support un polymère formant support (9) avec possibilité de liaison pour l'élément formant sonde (10).

13. Procédé selon l'une des revendications 10 à 12, **caractérisé en ce qu'**on utilise un support (9) ayant une longueur prédéfinie.

14. Procédé selon l'une des revendications 10 à 13, **caractérisé en ce qu'**on surveille la longueur d'un plateau de force (25) qui est affecté au support (9).

15. Procédé selon l'une des revendications 10 à 14, **caractérisé en ce qu'**on évalue la différence entre la hauteur d'un plateau de force (24) de l'élément formant sonde (10) et la hauteur du plateau de force (25) du support (9).
